# EUROPÄISCHE PATENTANMELDUNG

(11) **EP 4 002 379 A1**
(43) Veröffentlichungstag der Anmeldung: **25.05.2022**
(21) Anmeldenummer: 21207358.9
(22) Anmeldetag: 10.11.2021
(51) Int. Cl.: G16H 20/17

(54) **SYSTEM UMFASSEND EINE VORRICHTUNG, VERFAHREN UND COMPUTERPROGRAMM ZUM KONTROLLIEREN DES VERABREICHENS EINES MEDIKAMENTS AN EINEN PATIENTEN**

(30) Priorität: 12.11.2020 DE 102020129891
(71) Anmelder: Drägerwerk AG & Co. KGaA, 23558 Lübeck (DE)
(72) Erfinder: Althoff, Hinrich, 23558 Lübeck (DE)

(57) **Zusammenfassung**

Ausführungsbeispiele beziehen sich auf eine Vorrichtung, ein System umfassend die Vorrichtung, ein Verfahren und ein Computerprogramm zum Kontrollieren des Verabreichens eines Medikaments an einen Patienten. Die Vorrichtung (10) umfasst eine Verarbeitungseinrichtung (11) konfiguriert zum Empfangen von Information betreffend Vitaldaten des Patienten (31), Generieren eines Kontrollsignals in Bezug auf einen Kontrollparameter zum Kontrollieren des Verabreichens eines Medikaments mit einer Medikamentenverabreichungseinheit (21) an den Patienten (31) auf Basis der empfangenen Information betreffend Vitaldaten des Patienten. Die Vorrichtung (10) umfasst weiterhin eine Schnittstelle (12), die zum Bereitstellen des Kontrollsignals an die Medikamentenverabreichungseinheit (21) konfiguriert ist.

## Beschreibung

Die vorliegende Erfindung bezieht sich auf ein System umfassend eine Vorrichtung, ein Verfahren und ein Computerprogramm zum Kontrollieren des Verabreichens eines Medikaments an einen Patienten, insbesondere aber nicht ausschließlich, auf ein Konzept zur automatisierten Kontrolle und Unterbrechung einer Medikamentenverabreichung an einen Patienten.

Medikamente können in verschiedenen Darreichungsformen verabreicht werden. Beispielsweise ist eine Verabreichung von Medikamenten mit einer Medikamentenverabreichungseinheit möglich. Auf der Intensivstation werden viele flüssige Medikamente mit Spritzen- oder Infusionspumpen verabreicht. In der Regel können Pumpen ausschließlich manuell gestartet und gestoppt werden. Bestimmte Medikamente, beispielsweise Katecholamine, können stark die Vitaldaten des Patienten, beispielsweise den Blutdruck, beeinflussen. Typischerweise wird die Dosisrate des Medikaments manuell so eingestellt, dass der Blutdruck in einem bestimmten Fenster bleibt. Dies geschieht unter Umständen mehrfach am Tag. Gleichzeitig können die Vitaldaten des Patienten über das Patientenmonitoring mittels eines Patientenmonitors kontinuierlich überwacht werden. Bei Über- oder Unterschreiten eines Grenzwerts kann der Patientenmonitor alarmieren. Weiterhin kann mit einem Urimeter eine kontinuierliche Messung ermöglicht werden. Hierbei kann eine Ausscheidung von Urin als Vitalparameter verwendet werden. Ferner kann auch eine kontinuierliche Messung von Glukose im Blut als ein Vitalparameter in Verbindung mit einer Insulin-Gabe überwacht wer-den.

Zur Verabreichung von Medikamenten werden auch TCI -Pumpen (von engl. Target Controlled Infusion, zielgesteuerte Infusion) eingesetzt. Bei TCI-Pumpen zur zielgesteuerten Infusion für die Anästhesie kann die Dosisrate der Pumpe durch Software gesteuert und kontinuierlich gemäß pharmakokinetischer Modelle angepasst werden. TCI kann die Dosierung intravenöser Medikamente während der Operation automatisieren. Nachdem der Anästhesist die gewünschten Parameter am Gerät eingestellt und die Starttaste gedrückt hat, steuert die Infusionspumpe die Förderrate, während die Gabe vom Anästhesisten überwacht wird. TCI kann so sicher und effektiv wie manuell gesteuerte Infusionen sein. TCI-Pumpen wurden ursprünglich für die Dosierung von Propofol entwickelt. Hierbei wählt der Anästhesist lediglich eine gewünschte Zielkonzentration des Medikaments im Plasma aus. Die Förderrate wird dann durch einen Algorithmus in der Pumpe berechnet und über einen Zeitverlauf eingestellt. Der Algorithmus basiert auf einem pharmakokinetischen Modell, das den zeitlichen Verlauf der physiologischen Vorgänge von der intravenösen Gabe des Medikaments bis zur Plasmakonzentration abbildet. Die Modelle sind verhältnismäßig einfach und beziehen typischerweise das manuell eingegebene Gewicht, Größe und Geschlecht des Patienten ein, nicht aber aktuelle Messwerte oder Vitaldaten.

Eine wissenschaftliche Veröffentlichungen, "Target controlled infusion" (TCI) - ein Konzept mit Zukunft? Standortbestimmung, Handlungsempfehlungen und Blick in die Zukunft, S. Schraag, S. Kreuer, J. Bruhn, C. Frenkel, S. Albrecht; Der Anaesthesist; März 2008 bezieht sich auf TCI. Für weitere Details einer Anwendung einer Spritzenpumpe kann beispielsweise eine Gebrauchsanweisung einer Spritzenpumpe unter https://www.bd.com/documents/international/gu ides/directions-foruse/infusion/1000DF00331.pdf, abgerufen werden. Hier wird der Gebrauch der Spritzen-pumpe nach Model 80053UN01 des Herstellers beschrieben.

Nachteile der konventionellen Pumpen bestehen darin, dass die Dosisratenanpassung auf statistischen und statischen Modellen basiert. Außerdem sind TCI-Pumpen bisher nur für die Anwendung unter kontinuierlicher Aufsicht durch einen Anästhesisten zugelassen. Ein Problem liegt darin, dass die kontinuierliche Medikamentengabe durch Spritzenpumpen auf Intensivstationen ausschließlich manuell gestartet und gestoppt wird.

### Zusammenfassung

Vor diesem Hintergrund besteht daher ein Bedarf das Verabreichen eines Medikaments in-dividuell an einen Patienten/Patientenzustand anzupassen. Diesem Bedarf kommen die Ge-genstände der anhängigen unabhängigen Ansprüche nach.

Ausführungsbeispiele basieren auf dem Kerngedanken ein Verabreichen eines Medikaments mit einer Medikamentenverabreichungseinheit an einen Patienten auf Basis von empfangenen Information betreffend Vitaldaten des Patienten zu kontrollieren. Hierbei kann ein Kontrollparameter beispielsweise angeben, ob und wie eine Dosisrate zum Verabreichen des Medikaments erhöht werden kann, gesenkt werden kann, unverändert bleiben kann oder das Verabreichen des Medikaments unterbrochen werden kann. Beispielsweise kann die Verabreichung eines Medikaments auf Basis von kritischen Vitaldaten unterbrochen werden. Weiterhin kann beispielsweise eine automatische Dosisanpassung oder Unterbrechung der Gabe oder des Verabreichen des Medikaments stattfinden, wenn der Patient sich messbar in einem klinisch kritischen Bereich befindet oder sich dorthin entwickelt.

Ausführungsbeispiele des erfindungsgemäßen Systems schaffen eine Vorrichtung zum Kontrollieren eines Verabreichens eines Medikaments an einen Patienten. Die Vorrichtung umfasst eine Verarbeitungseinrichtung konfiguriert zum kontinuierlichen Empfangen von Information betreffend Vitaldaten des Patienten. Die Verarbeitungseinrichtung ist ferner ausgebildet zum Generieren eines Kontrollsignals in Be-zug auf einen Kontrollparameter zum Kontrollieren des Verabreichens eines Medikaments mit einer Medikamentenverabreichungseinheit an den Patienten auf Basis der empfangenen Information betreffend Vitaldaten des Patienten. Die Vorrichtung umfasst weiterhin eine Schnittstelle, die zum Bereitstellen des Kontrollsignals an die Medikamentenverabreichungseinheit konfiguriert ist. Hierbei kann der Kontrollparameter beispielsweise angeben, ob und wie eine Dosisrate zum Verabreichen des Medikaments erhöht werden kann, gesenkt werden kann, unverändert bleiben kann oder dass das Verabreichen des Medikaments unterbrochen wird. Dies kann ein einfaches und zuverlässiges Kontrollieren des Verabreichens des Medikaments an den Patienten in Abhängigkeit von den Vitaldaten des Patienten ermöglichen.

Hierbei können die Information betreffend Vitaldaten des Patienten ein Alarmsignal, dass Vitaldaten des Patienten in einem kritischen Bereich sind, umfassen. In diesem Fall kann die Unterbrechung der Verabreichung des Medikaments an den Patienten von Alarmen abhängig gemacht werden.

Dabei kann das Alarmsignal, einen Alarmzustand hoch, der angibt, dass Vitaldaten des Patienten über eine obere Alarmgrenze steigen, und/oder einen Alarmzustand niedrig, der an-gibt, dass Vitaldaten des Patienten über eine untere Alarmgrenze fallen, umfassen. Auf die-se Weise kann festgestellt werden, ob die obere Alarmgrenze überschritten oder die untere Alarmgrenze unterschritten wurde.

Beispielsweise kann die Verarbeitungseinrichtung weiterhin konfiguriert sein, der Medikamentenverabreichungseinheit den Kontrollparameter auf Basis der Information betreffend Vitaldaten des Patienten bereitzustellen. Der Kontrollparameter kann dabei angeben, ob und wie eine Dosisrate zum Verabreichen des Medikaments erhöht werden kann, gesenkt wer-den kann, unverändert bleiben kann oder das Verabreichen des Medikaments unterbrochen werden kann. Mit dem Kontrollparameter kann die Dosisrate zuverlässig kontrolliert werden und individuell für den Patienten eingestellt werden.

Weiterhin kann das von der Verarbeitungseinrichtung generierte Kontrollsignal mehrere Kontrollparameter umfassen. Jeder einzelne Kontrollparameter kann zum Kontrollieren des Verabreichens eines dem jeweiligen Kontrollparameter zugeordneten Medikaments an den Patienten ausgebildet sein. In diesem Fall kann das Verabreichen von mehreren Medikamenten an den Patienten zuverlässig kontrolliert werden.

In einigen Ausführungsbeispielen kann die Verarbeitungseinrichtung ferner konfiguriert sein, um den Kontrollparameter auf Basis der empfangenen Information betreffend Vitaldaten von zumindest zwei verschiedenen Vitalparametern des Patienten zu generieren. In diesem Fall können verschiedene Vitaldaten des Patienten einbezogen werden, um das Verabreichen des Medikaments an den Patienten zu kontrollieren. Hierbei kann die Verabreichung des Medikaments an den Patienten noch weiter verbessert und noch individueller an die jeweiligen Situation oder den Zustand des Patienten angepasst werden.

In weiteren Ausführungsbeispielen kann die Verarbeitungseinrichtung weiterhin konfiguriert sein, um den Kontrollparameter als Stellwert in einer Regelung bereitzustellen, wobei die Information betreffend Vitaldaten des Patienten als Messwert in der Regelung fungieren und die Regelung einen Integralregelanteil aufweist. Dies kann eine Regelung mit einer hohen Genauigkeit, insbesondere auch mit einer stationären Genauigkeit, ermöglichen.

Das erfindungsgemäße System umfasst neben der Vorrichtung ferner die Medikamentenverabreichungseinheit. Hierdurch kann die Verabreichung eines Medikaments mit dem System einfach und zuverlässig kontinuierlich kontrolliert werden.

Hierbei kann die Medikamentenverabreichungseinheit in Form einer Spritzenpumpe und/oder einer Infusionspumpe ausgebildet sein. Hierdurch kann das Medikament zuverlässig an den Patienten verabreicht werden.

Weiterhin umfasst das System erfindungsgemäß ein Vitaldatenmessgerät. Dadurch kann die Funktionalität des Systems erweitert werden und damit die Verabreichung des Medikaments an den Patienten noch einfacher kontinuierlich kontrolliert werden.

Dabei kann das Vitaldatenmessgerät ein Patientenmonitor und/oder ein Urimeter sein. Der Patientenmonitor und/oder das Urimeter kann dem Pflegepersonal die Möglichkeit bieten, die Vitaldaten des Patienten und gegebenenfalls weitere Daten schnell und übersichtlich zu erfassen.

Besonders bevorzugt ist die Medikamentenverabreichungseinheit zum Verabreichen von Katecholaminen und das Vitaldatenmessgerät zum Messen des Blutdrucks des Patienten ausgebildet.

In einigen Ausführungsbeispielen kann das Vitaldatenmessgerät ausgebildet sein, um das Alarmsignal in Bezug auf einen Alarmparameter zu generieren, der angibt, ob die Information betreffend Vitaldaten des Patienten einen vorgegebenen Grenzwert unter- oder über-schreiten. Dies kann dem Pflegepersonal ermöglichen schnell und zuverlässig zu erkennen, ob die Vitaldaten des Patienten den vorgegebenen Grenzwert unter- oder überschreiten.

In weiteren Ausführungsbeispielen können die Vorrichtung, die Medikamentenverabreichungseinheit und/oder das Vitaldatenmessgerät ausgebildet sein, um mittels eines gemein-samen Kommunikationsprotokolls in einem Netzwerk zu kommunizieren. In diesem Fall kann die Kommunikation der Vorrichtung, der Medikamentenverabreichungseinheit und/oder des Vitaldatenmessgerät zuverlässiger gestaltet werden und weiter vereinfacht werden.

Ausführungsbeispiele schaffen weiterhin ein Verfahren zum Kontrollieren des Verabreichens eines Medikaments an einen Patienten. Das Verfahren umfasst kontinuierliches Empfangen von In-formation betreffend Vitaldaten des Patienten, und Generieren eines Kontrollsignals in Be-zug auf einen Kontrollparameter zum Kontrollieren des Verabreichens eines Medikaments mit einer Medikamentenverabreichungseinheit an den Patienten auf Basis der Information betreffend Vitaldaten des Patienten. Hierbei kann das Verabreichens des Medikaments in Abhängigkeit von den Vitaldaten des Patienten kontrolliert werden.

Ein weiteres Ausführungsbeispiel ist ein Computerprogramm mit einem Programmcode zur Durchführung eines der hierin beschriebenen Verfahren, wenn der Programmcode auf einem Computer, einem Prozessor oder einer programmierbaren Hardwarekomponente ausgeführt wird. Ein maschinenlesbarer Datenträger mit einem solchen Programmcode ist ein weiteres Ausführungsbeispiel.

### Figurenkurzbeschreibung

Einige Beispiele von Vorrichtungen und/oder Verfahren werden nachfolgend bezugnehmend auf die beiliegenden Figuren lediglich beispielhaft näher erläutert. Es zeigen:
Fig. 1 ein Blockschaltbild eines Ausführungsbeispiels einer Vorrichtung zum Kontrollieren eines Verabreichens eines Medikaments an einen Patienten;
Fig. 2 ein Blockschaltbild eines Ausführungsbeispiels eines Systems umfassend die Vor-richtung;
Fig. 3 ein Blockschaltbild eines Ausführungsbeispiels eines weiteren Systems umfassend die Vorrichtung; und
Fig. 4 ein Blockschaltbild eines Ausführungsbeispiels eines Verfahrens zum Kontrollieren des Verabreichens eines Medikaments an einen Patienten.

Verschiedene Beispiele werden nun ausführlicher Bezug nehmend auf die beiliegenden Figuren beschrieben. In den Figuren können die Stärken von Linien, Schichten und/oder Be-reichen zur Verdeutlichung übertrieben sein.

Weitere Beispiele können Modifikationen, Entsprechungen und Alternativen abdecken, die in den Rahmen der Offenbarung fallen. Gleiche oder ähnliche Bezugszeichen beziehen sich in der gesamten Beschreibung der Figuren auf gleiche oder ähnliche Elemente, die bei einem Vergleich miteinander identisch oder in modifizierter Form implementiert sein können, während sie die gleiche oder eine ähnliche Funktion bereitstellen.

Es versteht sich, dass, wenn ein Element als mit einem anderen Element "verbunden" oder "gekoppelt" bezeichnet wird, die Elemente direkt, oder über ein oder mehrere Zwischenelemente, verbunden oder gekoppelt sein können. Wenn zwei Elemente A und B unter Ver-wendung eines "oder" kombiniert werden, ist dies so zu verstehen, dass alle möglichen Kombinationen offenbart sind, d. h. nur A, nur B sowie A und B, sofern nicht explizit oder implizit anders definiert. Eine alternative Formulierung für die gleichen Kombinationen ist "zumindest eines von A und B" oder "A und/oder B". Das Gleiche gilt, mutatis mutandis, für Kombinationen von mehr als zwei Elementen.

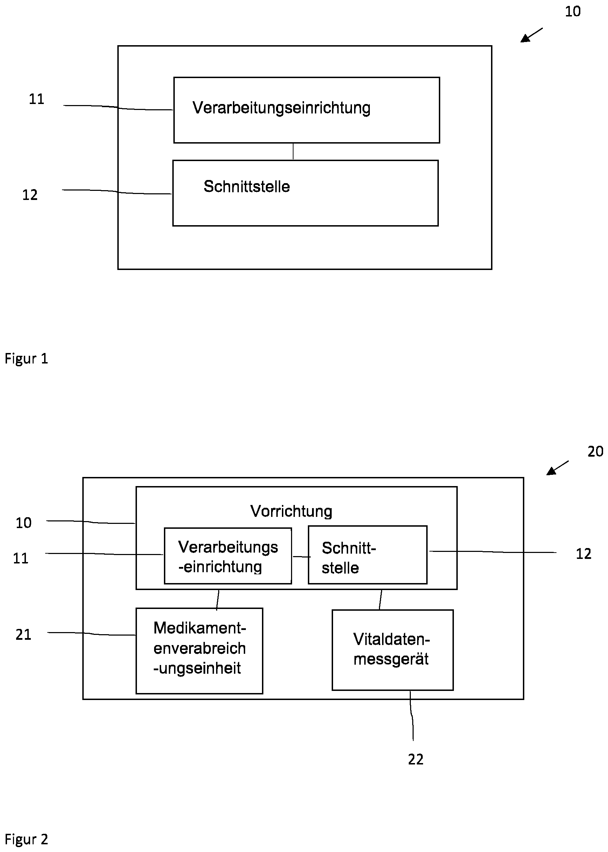

Fig. 1 zeigt ein Blockschaltbild eines Ausführungsbeispiels einer Vorrichtung 10 zum Kontrollieren eines Verabreichens eines Medikaments an einen Patienten. Die Vorrichtung 10 umfasst eine Verarbeitungseinrichtung 11 konfiguriert zum Empfangen von Information betreffend Vitaldaten des Patienten. Die Verarbeitungseinrichtung 11 ist ferner ausgebildet zum Generieren eines Kontrollsignals in Bezug auf einen Kontrollparameter zum Kontrollieren des Verabreichens eines Medikaments mit einer Medikamentenverabreichungseinheit an den Patienten auf Basis der empfangenen Information betreffend Vitaldaten des Patienten. Die Verarbeitungseinrichtung 11 kann in Form eines Prozessors, insbesondere in Form eines System-on-a-Chip, ausgebildet sein.

Die Verarbeitungseinrichtung 11 kann in Ausführungsbeispielen ein oder mehrere beliebige Kontroller, Mikrokontroller, Netzwerkprozessoren, Prozessorkerne, wie Digitale Signal Prozessorkerne (DSPs), programmierbare Hardwarekomponenten, usw. umfassen. Ausführungsbeispiele sind dabei nicht auf einen bestimmten Typ von Prozessorkern eingeschränkt. Es sind beliebige Prozessorkerne oder auch mehrere Prozessorkerne oder Mikrokontroller zur Implementierung einer Verarbeitungseinrichtung 11 denkbar. Es sind auch Implementierungen in integrierter Form mit anderen Vorrichtungen denkbar, beispielsweise in einer Steuereinheit die zusätzlich noch ein oder mehrere andere Funktionen umfasst. In Ausführungsbei-spielen kann eine Verarbeitungseinrichtung 11 durch einen Prozessorkern, einen Computerprozessorkern (CPU = Central Processing Unit), einen Grafikprozessorkern (GPU = Graphics Processing Unit), einen anwendungsspezifischen integrierten Schaltkreiskern (ASIC = Application-Specific Integrated Circuit), einen integrierten Schaltkreis (IC = Integrated Circuit), ein Ein-Chip-Systemkern (SOC = System on Chip), ein programmierbares Logikelement oder ein feldprogrammierbares Gatterarray mit einem Mikroprozessor (FPGA = Field Programmable Gate Array) als Kern des Bausteins oder der Bausteine realisiert sein.

Beispiele für Vitaldaten des Patienten können eine Atemfrequenz, ein Blutdruck, eine Sauerstoffsättigung und/oder eine Herzfrequenz des Patienten sein. Der Kontrollparameter kann beispielsweise angeben, ob und wie eine Dosisrate zum Verabreichen des Medikaments er-höht wird, gesenkt wird, unverändert bleibt oder das Verabreichen des Medikaments unterbrochen wird. Ein Fenster, in welchem sich der Vitalparameter befinden soll, kann von einem Anwender beispielsweise durch eine ärztliche Anordnung vorgegeben beziehungsweise eingestellt werden. Das Fenster kann durch eine obere Alarmgrenze und eine untere Alarmgrenze begrenzt werden. Der Anwender kann ein Arzt und/oder Pflegepersonal sein. Hierzu kann die Vorrichtung und/oder ein System ein User Interface, auch Benutzerschnittstelle genannt, umfassen. Das User Interface kann beispielsweise als Display, Touchscreen, Tastatur und/oder Mouse ausgebildet sein. Die Medikamentenverabreichungseinheit kann beispielsweise in Form einer Spritzenpumpe und/oder einer Infusionspumpe ausgebildet sein. Unter einer Spritzenpumpe versteht man eine Dosierpumpe zur kontinuierlichen parenteralen Verabreichung von Medikamenten. Unter einer Infusionspumpe, auch volumetrischen Pumpe genannt, versteht man eine Dosierpumpe zur kontinuierlichen, überwiegend intravenösen Verabreichung von Infusionen. Ferner können auch andere Medikamentenverabreichungs-einheiten verwendet werden.

Die Vorrichtung 10 umfasst weiterhin eine Schnittstelle 12, die zum Bereitstellen des Kontrollsignals an die Medikamentenverabreichungseinheit konfiguriert ist und die mit der Verarbeitungseinrichtung 11 gekoppelt ist. Die Schnittstelle 12 kann beispielsweise in Form einer Maschinenschnittstelle oder in Form einer Softwareschnittstelle ausgebildet sein.

Die Schnittstelle 12 kann in Ausführungsbeispielen als typische Schnittstelle zur Kommunikation in Netzwerken oder zwischen Netzwerkkomponenten oder medizinischen Geräten ausgebildet sein. Beispielsweise kann diese in Ausführungsbeispielen durch entsprechende Kontakte ausgebildet sein. Sie kann in Ausführungsbeispielen auch als separate Hardware ausgeführt sein und einen Speicher umfassen, der die zu sendenden oder die empfangenen Signale zumindest vorübergehend speichert. Die Schnittstelle 12 kann zum Empfang von elektrischen Signalen ausgebildet sein, zum Beispiel als Busschnittstelle, als optische Schnittstelle, als Ethernet-Schnittstelle, als Funkschnittstelle, als Feldbusschnittstelle, usw. Sie kann darüber hinaus in Ausführungsbeispielen zur Funkübertragung ausgebildet sein und ein Radio-Frontend sowie zugehörige Antennen umfassen.

Die Information betreffend Vitaldaten des Patienten kann ein Alarmsignal, dass Vitaldaten des Patienten in einem kritischen Bereich sind, umfassen. Das Alarmsignal kann beispiels-weise drei Zustände, einen Alarmzustand hoch, dass Vitaldaten des Patienten zu hoch sind (Alarmzustand "hoch") und über eine obere Alarmgrenze steigen, einen Alarmzustand niedrig, dass Vitaldaten des Patienten zu niedrig sind (Alarmzustand "niedrig") und unter eine untere Alarmgrenze fallen, und einen Normalzustand, umfassen. Der Alarmzustand kann beispielsweise angeben, dass Vitaldaten des Patienten sich in einem klinisch kritischen Be-reich befinden, entweder oberhalb oder unterhalb eines klinisch unkritischen Fensters. Während der Normalzustand beispielsweise angeben kann, dass sich die Vitaldaten des Patienten nicht in einem klinisch kritischen Bereich befinden. Das Alarmsignal kann zusätzlich ein akustisches Signal, das ausgegeben wird, umfassen. Auf diese Weise kann beispielsweise die Verabreichung eines blutdrucksenkenden Medikaments nur unterbrochen werden, wenn der gemessene Blutdruck unter die untere Alarmgrenze fällt, aber nicht, wenn der Blutdruck über die obere Alarmgrenze steigt.

Weiterhin kann die Verarbeitungseinrichtung 11 konfiguriert sein, um der Medikamentenverabreichungseinheit den Kontrollparameter auf Basis der Information betreffend Vitaldaten des Patienten bereitzustellen. Hierbei können der Verarbeitungseinrichtung 11 die Vital-daten des Patienten und/oder daraus abgeleitete Größen, die über die drei Zustände Alarm-zustand "hoch", Alarmzustand "niedrig" und Normalzustand hinausgehen, als Information betreffend Vitaldaten des Patienten gesendet werden. Hierbei kann der Kontrollparameter angeben, ob und wie eine Dosisrate zum Verabreichen des Medikaments erhöht wird, gesenkt wird, unverändert bleibt oder das Verabreichen des Medikaments unterbrochen wird.

Ferner kann das von der Verarbeitungseinrichtung 11 generierte Kontrollsignal mehrere Kontrollparameter umfassen. Dabei kann jeder einzelne Kontrollparameter zum Kontrollieren des Verabreichens eines dem jeweiligen Kontrollparameter zugeordneten Medikaments an den Patienten ausgebildet sein. Hierbei kann das Verabreichen mehrerer Medikamente an den Patienten kontrolliert werden. Dabei kann jeder Kontrollparameter in Bezug auf das ihm zugeordnete Medikament beispielsweise angeben, ob und wie eine Dosisrate zum Verabreichen des Medikaments erhöht wird, gesenkt wird, unverändert bleibt oder das Verabreichen des Medikaments unterbrochen wird.

In einem einfachen Ausführungsbeispiel liegt beispielsweise ein Vitalwert eines Patienten außerhalb eines vordefinierten Bereichs (z.B. Blutdruck zu niedrig). Daraufhin wird eine diesbezügliche Information empfangen, z.B. einen diesen Zusammenhang anzeigenden Indikator oder ein Bit/Flag. Nunmehr wird die Verabreichung eines blutdrucksenkenden Medikaments unterbrochen, was der Medikamentenverabreichungseinheit von der Verarbeitungseinrichtung 11 durch das Kontrollsignal angezeigt wird. Das Kontrollsignal kann dann ebenfalls einen einfachen Indikator, ein Bit, ein Flag oder ein Interruptsignal (Unterbrechungssignal) umfassen.

Die Verarbeitungseinrichtung 11 kann weiterhin konfiguriert sein, um den Kontrollparameter auf Basis der empfangenen Information betreffend Vitaldaten von zumindest zwei verschiedenen Vitalparametern des Patienten zu generieren. Hierbei können verschiedene Vitaldaten wie beispielsweise Atemfrequenz, Blutdruck, Sauerstoffsättigung und/oder Herz-frequenz in das Generieren des Kontrollparameters für das Verabreichen eines Medikaments einfließen.

Weiterhin kann die Verarbeitungseinrichtung 11 konfiguriert sein, um den Kontrollparameter als Stellwert in einer Regelung bereitzustellen. Hierbei können die Information betreffend Vitaldaten des Patienten als Messwert in der Regelung fungieren und die Regelung einen Integralregelanteil aufweisen. Der Integralregelanteil kann eine stationäre Genauigkeit aufweisen. Weiterhin kann die Regelung einen Proportionalregelanteil und/oder einen Differenzialregelanteil aufweisen.

Das in Fig. 1 abgebildete Ausführungsbeispiel kann ein oder mehrere zusätzliche optionale Merkmale umfassen.

Fig. 2 zeigt ein Blockschaltbild eines Ausführungsbeispiels eines Systems 20 umfassend die Vorrichtung 10. Das System 20 umfasst die zuvor beschriebene Vorrichtung 10 und die Medikamentenverabreichungseinheit 21. Die Medikamentenverabreichungseinheit 21 kann in Form einer Spritzenpumpe und/oder einer Infusionspumpe ausgebildet sein. Unter einer Spritzenpumpe versteht man eine Dosierpumpe zur kontinuierlichen parenteralen Verabreichung von Medikamenten. Unter einer Infusionspumpe, auch volumetrischen Pumpe genannt, versteht man eine Dosierpumpe zur kontinuierlichen, überwiegend intravenösen, Verabreichung von Infusionen. Ferner können auch andere Medikamentenverabreichungseinheiten verwendet werden.

Das System 20 kann weiterhin ein Vitaldatenmessgerät 22 umfassen. Das Vitaldatenmessgerät 22 kann ein Patientenmonitor sein. Patientenmonitore können zum Messen und Überwachen von Vitaldaten des Patienten dienen. Patientenmonitore können beispielsweise während der Narkose bei Operationen, bei kritisch kranken Patienten in der Intensivmedizin, während Untersuchungen mit einer Sedierung oder bei anderen Krankheitsbildern, die eine kontinuierliche Überwachung erfordern, eingesetzt werden. Für den Einsatz bei Notfallpatienten kann der Patientenmonitor auch als mobiles Gerät ausgebildet sein.

Hierbei kann das Vitaldatenmessgerät 22 ausgebildet sein, um das Alarmsignal in Bezug auf einen Alarmparameter zu generieren, der angibt, ob die Information betreffend Vitaldaten des Patienten einen vorgegebenen Grenzwert unter- oder überschreiten. Dabei kann die Medikamentenverabreichungseinheit 21 oder Pumpenabschaltung einen separaten Grenzwert verwenden, der von dem Grenzwert oder dem Alarmgrenzwert des Vitaldatenmessgeräts 22 oder des Patientenmonitors abweicht. Die Eigenschaften bezüglich der Alarmierung können beispielsweise beim Patientenmonitor unverändert zu konventionellen Methoden bleiben.

Ferner können die Vorrichtung 10, die Medikamentenverabreichungseinheit 21 und/oder das Vitaldatenmessgerät 22 ausgebildet sein, um mittels eines gemeinsamen Kommunikations-protokolls in einem Netzwerk zu kommunizieren. Als gemeinsames Kommunikationsprotokoll kann beispielsweise auch IEE 11073 SDC (von engl. Service-oriented Device Connectivity, dienstorientierte Geräteverbindung) verwendet werden. Es können auch andere Kommunikationsprotokolle verwendet werden.

Die IEEE (Institute of Electrical and Electronics Engineers) 11073 SDC Standardfamilie definiert ein Kommunikationsprotokoll für Medizingeräte, deren Einsatzbereich in der potenziell kritischen Patientenversorgung liegt, wie etwa dem Operationssaal, der Intensivstation, aber auch in anderen Bereichen des Krankenhauses. Sie ist Teil der ISO/IEEE 11073 Normenfamilie. IEEE 11073 SDC basiert auf dem Paradigma der serviceorientierten Architektur, SOA. Die Einführung von SDC als Standard zur bidirektionalen Kommunikation von Mess- und Einstellwerten zwischen Medizingeräten kann auch Möglichkeiten der Fernsteuerung eröffnen. Closed-Loop-Systeme insbesondere für die Steuerung von Medikamentengaben mit Spritzenpumpen können sehr aufwändig bezüglich ihrer Validierung sein, da große Studien erforderlich sein können. Ein sinnvoller Zwischenschritt können automatische Notfallabschaltungen sein, die helfen können, kritische Situationen zu vermeiden.

Das in Fig. 2 abgebildete Ausführungsbeispiel kann ein oder mehrere zusätzliche optionale Merkmale umfassen.

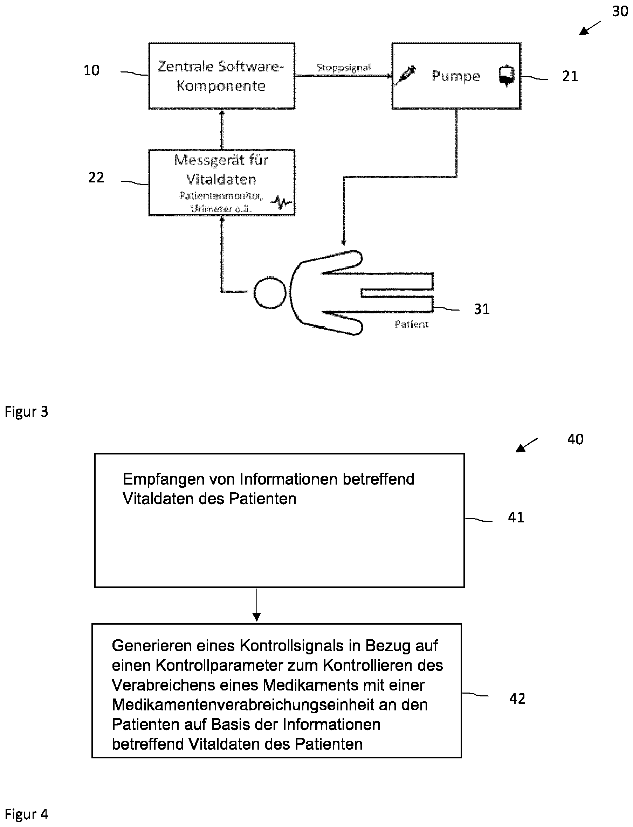

Fig. 3 zeigt ein Blockschaltbild eines Ausführungsbeispiels eines weiteren Systems 30 umfassend die Vorrichtung 10. Das System 30 kann ähnlich implementiert werden wie das System 20, das in Verbindung mit Fig. 2 beschrieben ist. Das System 30 umfasst die Vorrichtung 10, die Medikamentenverabreichungseinheit 21 und das Vitaldatenmessgerät 22. Die Vorrichtung 10 ist vorliegend als zentrale Software-Komponente ausgebildet, die auf entsprechender Hardware ausgeführt wird. Die Vorrichtung 10 ist implementiert, wie in Ver-bindung mit Fig. 1 beschrieben, und umfasst unter anderem die dort beschriebenen Verarbeitungseinrichtung 11 und Schnittstelle 12 als Hardwarekomponenten. Die Vorrichtung 10 ist mit dem Vitaldatenmessgerät 22 verbunden. Das Vitaldatenmessgerät 22 oder Messgerät für Vitaldaten ist zum Messen von Vitaldaten des Patienten 31 ausgebildet. Ferner kann das Vitaldatenmessgerät 22 auch zum, insbesondere gleichzeitigen, Messen von unterschiedlichen Vitaldaten wie beispielsweise Herzfrequenz, Atemfrequenz, Sauerstoffsättigung und/ oder Blutdruck des Patienten 31 ausgebildet sein.

Die Medikamentenverabreichungseinheit kann in diesem oder einem der weiteren genannten Ausführungsbeispielen zum Verabreichen von Katecholaminen ausgebildet sein. Vorzugsweise ist das Vitaldatenmessgerät gleichzeitig zum Messen des Blutdrucks des Patienten ausgebildet. Hierdurch kann das erfindungsgemäße System vorteilhaft für eine Katecholamintherapie eingesetzt werden.

Das Vitaldatenmessgerät 22 kann beispielsweise als Patientenmonitor und/oder Urimeter ausgebildet sein. Das Urimeter kann als ein steriles geschlossenes Sammelsystem mit integrierter Messkammer ausgebildet sein. Das Urimeter kann weiterhin ein vertikales Entwässerungssystem und eingebaute Rückschlagventile umfassen, die sicher stellen können, dass sich die Messkammer nach einer Messung vollständig entleeren kann, um genaue Messungen insbesondere bei geringem Urinausstoß gewährleisten zu können. Das Urimeter, das ähnlich wie ein Hydrometer ausgebildet sein kann, kann beispielsweise zur Messung eines spezifischen Gewichts oder eines Fluss eines Urins verwendet werden. Das spezifische Gewicht des Urins kann eine Funktion einer Anzahl, einer Dichte und eines Gewichts von im Urin vorhandenen gelösten Partikel sein und kann als Maß für eine Konzentrationskraft einer Niere verwendet werden.

Das Vitaldatenmessgerät 22 kann zum Messen der Vitaldaten des Patienten 31 mit dem Patienten 31 verbunden sein. Beispielsweise kann das Vitaldatenmessgerät 22 eine Manschette umfassen, die ausgebildet sein kann, um einen Arm des Patienten 31 zu umgeben und damit den Blutdruck des Patienten 31 bestimmen zu können. Weiterhin kann eine invasive, arterielle Blutdruckmessung erfolgen. Die invasiven Blutdruckmessung, IBP, ist eine Form der Blutdruckmessung, bei der ein Messfühler über einen arteriellen Zugang direkt in eine Arterie des Patienten eingeführt wird. Die Arterie kann mit einer Kanüle punktiert und an ein flüssigkeitsgefülltes Schlauchsystem angeschlossen werden. Eine Druckveränderungen innerhalb der Arterie kann im flüssigkeitsgefüllten Schlauchsystem an einen Druckumwandler, auch Transducer genannt, weitergeleitet werden. Dort kann eine Druckwelle von einer Membran registriert und in elektrische Impulse umgewandelt werden, die von einem angeschlossenen Messgerät angezeigt werden können. Weiterhin kann das Vitaldatenmess-gerät 22 ein Herzfrequenzmessgerät beispielsweise in Form eines Brustgurts umfassen und/oder Hautelektroden umfassen, um damit die Herzfrequenz des Patienten 31 bestimmen zu können. Das Vitaldatenmessgerät 22 kann Information betreffend der Vitaldaten des Patienten 31 beispielsweise über eine Luftschnittstelle oder eine Kabelverbindung an die Vorrichtung 10 senden. Die Vorrichtung 10 generiert ein Kontrollsignal in Bezug auf einen Kontrollparameter zum Kontrollieren des Verabreichens eines Medikaments mit der Medikamentenverabreichungseinheit 21 an den Patienten 31 auf Basis der Information betreffend Vitaldaten des Patienten 31.

Die Vorrichtung 10 kann hierbei ein Stoppsignal als Kontrollsignal generieren, wenn Information betreffend Vitaldaten des Patienten 31 anzeigen, dass die Vitaldaten des Patienten 31 sich in einem klinisch kritischen Bereich befinden. Das Stoppsignal kann beispielsweise einen Alarmzustand hoch, dass Vitaldaten des Patienten zu hoch sind (Alarmzustand "hoch"), und einen Alarmzustand niedrig, dass Vitaldaten des Patienten zu niedrig sind (Alarmzustand "niedrig"), umfassen. Der Alarmzustand kann beispielsweise angeben, dass Vitaldaten des Patienten sich in einem klinisch kritischen Bereich befinden, entweder ober-halb oder unterhalb eines klinisch unkritischen Fensters. In diesem Fall kann die Vorrichtung 10 das Stoppsignal an die Medikamentenverabreichungseinheit 21 senden, um das Verabreichen des Medikaments an den Patienten 31 mit der Medikamentenverabreichungseinheit 21 zu unterbrechen.

Zum Beispiel kann die Medikamentengabe einer Spritzenpumpe unterbrochen werden, wenn ein vom Medikament beeinflusster Vitalparameter einen eingestellten Grenzwert über- oder unterschreitet. Dazu kann beispielsweise eine zentrale Instanz im Netzwerk wie die Vorrichtung 10 die Alarme eines Patientenmonitors überwachen. Gleichzeitig kann die Instanz über Information darüber verfügen, welche Spritzenpumpen mit welchen Medikamenten an denselben Patienten 31 angeschlossen sind. Sobald der entsprechende Alarm am Gerät oder Vitaldatenmessgerät 22 anliegt und beispielsweise per SDC ins Netzwerk kommuniziert wird, kann die Instanz einen Fernsteuerungsbefehl an die entsprechende Spritzenpumpe oder Medikamentenverabreichungseinheit 21 senden, um die Gabe zu unterbrechen. In einer für den Patienten 31 kritischen Situation kann dann nicht nur alarmiert werden, sondern auch automatisch eine möglicherweise ursächliche oder mindestens kontraindizierte Medikamentengabe unterbrochen werden. Eine Voraussetzung dafür kann sein, dass Pumpen Fernsteuerbarkeit erlauben, um die Gabe zu unterbrechen.

Gemäß einem Ausführungsbespiel kann der Patient 31 auf der Intensivstation Dobutamin erhalten, um einen zu niedrigen Blutdruck des Patienten 31 zu behandeln. Die Alarmgrenzen des Patientenmonitors können dabei relativ eng eingestellt sein, um einerseits einen weiteren Blutdruckabfall trotz der Medikation frühzeitig zu erkennen, andererseits, um bei einem steigenden Blutdruck in einen für den Patienten 31 kritischen Bereich die Medikamentengabe abzubrechen. Sollte nun der Blutdruck aufgrund pathophysiologischer Umstände z.B. über die Alarmgrenze von 140 mmHg Mitteldruck steigen, alarmiert der Patientenmonitor entsprechend und kommuniziert den Alarm ins Netzwerk. Dort empfängt die zentrale Instanz den Alarm und sendet an die Medikamentenverabreichungseinheit 21 oder eine Dobutamin-Pumpe den Befehl die Zufuhr zu stoppen. Eine Dobutamin-Pumpe kann der Abgabe von Dobutamin an den Patienten 31 dienen.

Das in Fig. 3 abgebildete Ausführungsbeispiel kann ein oder mehrere zusätzliche optionale Merkmale umfassen.

Fig. 4 zeigt ein Blockschaltbild eines Ausführungsbeispiels eines Verfahrens 40 zum Kontrollieren des Verabreichens eines Medikaments an einen Patienten 31. Das Verfahren 40 umfasst Empfangen 41 von Information betreffend Vitaldaten des Patienten. Das Verfahren 40 umfasst weiterhin Generieren 42 eines Kontrollsignals in Bezug auf einen Kontrollpara-meter zum Kontrollieren des Verabreichens eines Medikaments mit einer Medikamentenverabreichungseinheit an den Patienten 31 auf Basis der Information betreffend Vitaldaten des Patienten 31. Dabei kann der Kontrollparameter beispielsweise angeben, ob und wie eine Dosisrate zum Verabreichen des Medikaments erhöht wird, gesenkt wird, unverändert bleibt oder das Verabreichen des Medikaments unterbrochen wird. Hierbei kann die Dosisrate der Medikamentenverabreichungseinheit 21 oder einer Pumpe von der Vorrichtung 10 gesteuert werden. Die Dosisrate kann wahlweise auf einen voreingestellten Wert abgesenkt werden oder auf null abgesenkt werden. In einem Anwendungsfall kann die zentrale Instanz oder Vorrichtung 10 anhand von Regeln erkennen, dass die Dosisrate zu hoch oder zu niedrig ist in Bezug auf die Vitaldaten des Patienten 31 und die Gabe oder das Verabreichen des Medikaments abbrechen, um eine Fehlmedikation bezüglich des aktuellen Patientenzustands zu verhindern.

Gemäß einem weiteren Ausführungsbespiel kann mit einem Medikament ein Blutdruck des Patienten kontrolliert werden. Hierbei kann ein Medikamentenstopp, bei dem eine Verabreichung des Medikaments mit der Medikamentenverabreichungseinheit 21 gestoppt wird, aufgrund einer Herzfrequenz des Patienten ausgelöst werden. Dabei kann ein Sepsis-Patient 31 über die Medikamentenverabreichungseinheit 21 oder einen zentralen Venenkatheter Phenylephrin als Vasopressor erhalten, um den zu niedrigen Blutdruck zu behandeln. Der zentrale Venenkatheter, auch zentralvenöser Katheter genannt, kann als ein dünner Kunst-stoffschlauch ausgebildet sein, der über eine Vene einer oberen Körperhälfte in ein Venen-system eingeführt wird und dessen Ende in einer oberen oder unteren Hohlvene vor einem rechten Vorhof des Herzens liegen kann. Als Vasopressoren können Substanzen bezeichnet werden, welche dazu eingesetzt werden können, den Blutdruck zu heben oder zu stützen. Die Folgen sind ein erhöhter Gefäßtonus, eine erhöhte Kontraktilität des Herzens und ein erhöhtes kardiales Pumpvolumen. Das Medikament kann neben dem Blutdruck allerdings auch die Herzfrequenz beeinflussen und Bradykardien auslösen. Bradykardie ist ein verlangsamter Herzschlag und bezeichnet in der Medizin beim erwachsenen Menschen eine Herzschlagfrequenz unter 60 Schlägen pro Minute. Wenn die Herzfrequenz des Patienten 31 unter den eingestellten Grenzwert von z.B. 40 pro Minute fällt, kann die Gabe der Pumpe oder der Medikamentenverabreichungseinheit 21 gestoppt werden.

In einem weiteren Ausführungsbespiel kann der Patient 31 Flüssigkeit mit 150mL/h über eine Infusionspumpe oder die Medikamentenverabreichungseinheit 21 an einen zentralen Zugang oder den zentralen Venenkatheter erhalten. Die Infusionspumpe ist eine Dosier-pumpe zur kontinuierlichen, überwiegend intravenösen, Verabreichung von Infusionen. Wenn der zentrale Venendruck zu hoch wird, kann die Infusionspumpe gestoppt werden oder die Rate der Pumpe oder Infusionspumpe beispielsweise auf 50mL/h reduziert werden, siehe auch Ausführungsbespiel zur Behandlung des Patienten 31 mit Adrenalin.

Gemäß einem weiteren Ausführungsbeispiel kann der Patient 31 den Wirkstoff Furosemid erhalten, um die Diurese zu befördern. Furosemid ist ein Arzneistoff aus der Gruppe der Schleifendiuretika. Schleifendiuretika führen zur Ausscheidung großer Mengen von Gewebeflüssigkeit, indem sie in der Niere ein Transportprotein hemmen. Diurese bezeichnet die Harnausscheidung durch die Nieren. Ein Urimeter kann eine Ausscheidung und ein Erreichen einer bestimmten Menge oder einer Ausscheidungsrate messen, bei der die Furosemid-Gabe abgebrochen werden soll.

Ausführungsbeispiele können weiterhin ein Computerprogramm mit einem Programmcode zum Ausführen oder Durchführen eines oder mehrerer der obigen Verfahren sein oder sich darauf beziehen, wenn das Computerprogramm auf einem Computer, Prozessor oder einer programmierbaren Hardwarekomponente oder der Verarbeitungseinrichtung 11 ausgeführt wird. Schritte, Operationen oder Prozesse von verschiedenen, oben beschriebenen Verfahren können durch programmierte Computer oder Prozessoren ausgeführt werden. Beispiele können auch Programmspeichervorrichtungen, z. B. Digitaldatenspeichermedien, abdecken, die maschinen-, prozessor- oder computerlesbar sind und maschinenausführbare, prozessoraus-führbare oder computerausführbare Programme von Anweisungen codieren. Die Anweisungen führen einige oder alle der Schritte der oben beschriebenen Verfahren aus oder verursachen deren Ausführung. Die Programmspeichervorrichtungen können z. B. Digitalspeicher (Flash-Speicher oder Solid State Drive-Speicher), magnetische Speichermedien wie beispielsweise Magnetplatten und Magnetbänder, Festplattenlaufwerke oder optisch lesbare Digitaldatenspeichermedien umfassen oder sein. Weitere Beispiele können auch Computer, Prozessoren oder Steuereinheiten, die zum Ausführen der Schritte der oben beschriebenen Verfahren programmiert sind, oder (feld-) programmierbare Logik-Arrays ((F)PLAs = (Field) Programmable Logic Arrays) oder (feld-)programmierbare Gate-Arrays ((F)PGA = (Field) Programmable Gate Arrays), die zum Ausführen der Schritte der oben beschriebenen Verfahren programmiert sind, abdecken.

Das in Fig. 4 abgebildete Ausführungsbeispiel kann ein oder mehrere zusätzliche optionale Merkmale umfassen.

Funktionen verschiedener in den Figuren gezeigter Elemente sowie die bezeichneten Funktionsblöcke können in Form dedizierter Hardware, z. B "eines Signalanbieters", "einer Signalverarbeitungseinheit", "eines Prozessors", "einer Steuerung" etc. sowie als Hardware fähig zum Ausführen von Software in Verbindung mit zugehöriger Software implementiert sein. Bei Bereitstellung durch einen Prozessor können die Funktionen durch einen einzelnen dedizierten Prozessor, durch einen einzelnen gemeinschaftlich verwendeten Prozessor oder durch eine Mehrzahl von individuellen Prozessoren bereitgestellt sein, von denen einige oder von denen alle gemeinschaftlich verwendet werden können.

Allerdings ist der Begriff "Prozessor" oder "Steuerung" bei Weitem nicht auf ausschließlich zur Ausführung von Software fähige Hardware begrenzt, sondern kann Digitalsignalprozessor-Hardware (DSP-Hardware; DSP = Digital Signal Processor), Netzprozessor, anwendungs-spezifische integrierte Schaltung (ASIC = Application Specific Integrated Circuit), feldprogrammierbare Logikanordnung (FPGA = Field Programmable Gate Array), Nurlesespeicher (ROM = Read Only Memory) zum Speichern von Software, Direktzugriffsspeicher (RAM = Random Access Memory) und nichtflüchtige Speichervorrichtung (storage) umfassen. Sonstige Hardware, herkömmliche und/oder kundenspezifische, kann auch eingeschlossen sein.

Ein Blockdiagramm kann zum Beispiel ein grobes Schaltdiagramm darstellen, dass die Grundsätze der Offenbarung implementiert. Auf ähnliche Weise können ein Flussdiagramm, ein Ablaufdiagramm, ein Zustandsübergangsdiagramm, ein Pseudocode und dergleichen verschiedene Prozesse, Operationen oder Schritte repräsentieren, die zum Beispiel im Wesentlichen in computerlesbarem Medium dargestellt und so durch einen Computer oder Prozessor ausgeführt werden, ungeachtet dessen, ob ein solcher Computer oder Prozessor explizit gezeigt ist. In der Beschreibung oder in den Patentansprüchen offenbarte Verfahren können durch ein Bauelement implementiert werden, das ein Mittel zum Ausführen eines jeden der jeweiligen Schritte dieser Verfahren aufweist.

Es versteht sich, dass die Offenbarung mehrerer, in der Beschreibung oder den Ansprüchen offenbarter Schritte, Prozesse, Operationen oder Funktionen nicht als in der bestimmten Reihenfolge befindlich ausgelegt werden soll, sofern dies nicht explizit oder implizit anderweitig, z. B. aus technischen Gründen, angegeben ist. Daher werden diese durch die Offenbarung von mehreren Schritten oder Funktionen nicht auf eine bestimmte Reihenfolge begrenzt, es sei denn, dass diese Schritte oder Funktionen aus technischen Gründen nicht austauschbar sind. Ferner kann bei einigen Beispielen ein einzelner Schritt, Funktion, Prozess oder Operation mehrere Teilschritte, -funktionen, -prozesse oder -operationen einschließen und/oder in dieselben aufgebrochen werden. Solche Teilschritte können eingeschlossen sein und Teil der Offenbarung dieses Einzelschritts sein, sofern sie nicht explizit ausgeschlossen sind.

### Bezugszeichenliste

- 10: Vorrichtung;
- 11: Verarbeitungseinrichtung;
- 12: Schnittstelle;
- 20: System;
- 21: Medikamentenverabreichungseinheit;
- 22: Vitaldatenmessgerät;
- 30: System;
- 31: Patient;
- 40: Verfahren;
- 41: Empfangen von Information betreffend Vitaldaten des Patienten; und
- 42: Generieren eines Kontrollsignals in Bezug auf einen Kontrollparameter zum Kontrollieren des Verabreichens eines Medikaments.

## Patentansprüche

1. System (20, 30), umfassend
eine Vorrichtung (10) zum Kontrollieren eines Verabreichens eines Medikaments an einen Patienten (31), umfassend:
- eine Verarbeitungseinrichtung (11) konfiguriert zum:
kontinuierlichen Empfangen von Information betreffend Vitaldaten des Patienten (31) von einem Vitaldatenmessgerät,
Generieren eines Kontrollsignals in Bezug auf einen Kontrollparameter zum Kontrollieren des Verabreichens eines Medikaments mit einer Medikamentenverabreichungseinheit (21) an den Patienten (31) auf Basis der empfangenen Information betreffend Vitaldaten des Patienten (31), und
eine Schnittstelle (12), die zum Bereitstellen des Kontrollsignals an die Medikamentenverabreichungseinheit (21) konfiguriert ist;
- die Medikamentenverabreichungseinheit (21); und
- das Vitaldatenmessgerät (22), welches ausgebildet ist zum kontinuierlichen Überwachen der Vitaldaten des Patienten.

2. System (20, 30) gemäß Anspruch 1, wobei die Information betreffend Vitaldaten des Patienten (31) ein Alarmsignal, dass Vitaldaten des Patienten (31) in einem kritischen Bereich sind, umfasst.

3. System (20, 30) gemäß Anspruch 2, wobei das Alarmsignal, einen Alarmzustand hoch, der angibt, dass Vitaldaten des Patienten über eine obere Alarmgrenze steigen, und/oder einen Alarmzustand niedrig, der angibt, dass Vitaldaten des Patienten über eine untere Alarmgrenze fallen, umfasst.

4. System (20, 30) gemäß einem der vorhergehenden Ansprüche, wobei die Verarbeitungseinrichtung (11) konfiguriert ist, der Medikamentenverabreichungseinheit (21) den Kontrollparameter auf Basis der Information betreffend Vitaldaten des Patienten (31) bereitzustellen, wobei der Kontrollparameter angibt, ob und wie eine Dosisrate zum Verabreichen des Medikaments erhöht wird, gesenkt wird, unverändert bleibt oder das Verabreichen des Medikaments unterbrochen wird.

5. System (20, 30) gemäß einem der vorhergehenden Ansprüche, wobei das von der Verarbeitungseinrichtung (11) generierte Kontrollsignal mehrere Kontrollparameter umfasst, wobei jeder einzelne Kontrollparameter zum Kontrollieren des Verabreichens eines dem jeweiligen Kontrollparameter zugeordneten Medikaments an den Patienten (31) ausgebildet ist.

6. System (20, 30) gemäß einem der vorhergehenden Ansprüche, wobei die Verarbeitungseinrichtung (11) weiterhin konfiguriert ist, um den Kontrollparameter auf Basis der empfangenen Information betreffend Vitaldaten von zumindest zwei verschiedenen Vitalparametern des Patienten (31) zu generieren.

7. System (20, 30) gemäß einem der vorhergehenden Ansprüche, wobei die Verarbeitungseinrichtung (11) konfiguriert ist, um den Kontrollparameter als Stellwert in einer Regelung bereitzustellen, wobei die Information betreffend Vitaldaten des Patienten (31) als Messwert in der Regelung fungieren und die Regelung einen Integralregelanteil aufweist.

8. System (20, 30) gemäß einem der vorhergehenden Ansprüche, wobei die Medikamentenverabreichungseinheit (21) in Form einer Spritzenpumpe und/oder einer Infusionspumpe ausgebildet ist.

9. System (20, 30) gemäß einem der vorhergehenden Ansprüche, wobei die Medikamentenverabreichungseinheit zum Verabreichen von Katecholaminen und das Vitaldatenmessgerät zum Messen des Blutdrucks des Patienten ausgebildet ist.

10. System (20, 30) gemäß einem der vorhergehenden Ansprüche, wobei das Vitaldatenmessgerät (22) ausgebildet ist, um das Alarmsignal in Bezug auf einen Alarmparameter zu generieren, der angibt, ob die Information betreffend Vitaldaten des Patienten (31) einen vorgegebenen Grenzwert unter- oder überschreiten.

11. System (20, 30) gemäß einem der vorhergehenden Ansprüche, wobei die Vorrichtung (10), die Medikamentenverabreichungseinheit (21) und/oder das Vitaldatenmessgerät (22) ausgebildet sind, um mittels eines gemeinsamen Kommunikationsprotokolls in einem Netzwerk zu kommunizieren.

12. Verfahren (40) zum Kontrollieren des Verabreichens eines Medikaments an einen Patienten (31), umfassend:
kontinuierliches Empfangen von Information betreffend Vitaldaten des Patienten (31), und
Generieren eines Kontrollsignals in Bezug auf einen Kontrollparameter zum Kontrollieren des Verabreichens eines Medikaments mit einer Medikamentenverabreichungseinheit (21) an den Patienten (31) auf Basis der Information betreffend Vitaldaten des Patienten (31).

13. Computerprogramm mit einem Programmcode zur Durchführung eines der Ver-fahren (40) gemäß Anspruch 12, wenn der Programmcode auf einem Computer, einem Prozessor oder einer programmierbaren Hardwarekomponente ausgeführt wird.
